# EUROPEAN PATENT APPLICATION

(11) **EP 3 739 325 A1**
(43) Date of publication of application: **18.11.2020**
(21) Application number: 18899233.3
(22) Date of filing: 30.11.2018
(51) Int. Cl.: G01N 23/2055, G01L 1/25

(54) **RESIDUAL STRESS MEASURING METHOD**

(30) Priority: 12.01.2018 JP 2018003371
(71) Applicant: Kabushiki Kaisha Kobe Seiko Sho (Kobe Steel, Ltd.), Kobe-shi, Hyogo 651-8585 (JP)
(72) Inventor: MATSUDA Mariko, Takasago-shi, Hyogo 676-8670 (JP); KABUTOMORI Tatsuhiko, Takasago-shi, Hyogo 676-8670 (JP); TAKAMATSU Hiroyuki, Hyogo 651-2271 (JP)
(74) Representative: Müller-Boré & Partner Patentanwälte PartG mbB
(86) International application number: PCT/JP2018/044208
(87) International publication number: WO 2019/138727

(57) **Abstract**

The present invention is a method for measuring a residual stress in a cast and forged steel product, the method using X-rays, including: irradiating a cast and forged steel product with X-rays; two-dimensionally detecting intensities of diffracted X-rays originating from the X-rays; and calculating a residual stress based on a diffraction ring formed by an intensity distribution of the diffracted X-rays detected in the detecting, wherein the irradiating includes changing a condition for irradiation of the cast and forged steel product with the X-rays, the irradiating is a step of performing the changing each time the cast and forged steel product is irradiated with the X-rays, the calculating is a step of calculating the residual stress each time the cast and forged steel product is irradiated with the X-rays, and the method for measuring a residual stress further includes averaging a plurality of residual stresses calculated in the calculating after the irradiating, the detecting, and the calculating are performed in this order a plurality of times.

## Description

### [TECHNICAL FIELD]

The present invention relates to a method for measuring a residual stress.

### [BACKGROUND ART]

Recently, a residual stress measurement technique using X-rays has been widely applied. In this technique, a lattice distortion occurring inside a specimen having a crystalline structure is measured using X-rays, and the measurement result is converted into a residual stress.

As a method for measuring a residual stress using X-rays, the cos α method is known. In the cos α method, a specimen is irradiated with X-rays at a specific irradiation angle, intensities of diffracted X-rays generated by reflection of the X-rays by the specimen are two-dimensionally detected, and a residual stress is calculated based on a diffraction ring formed by an intensity distribution of the diffracted X-rays which have been detected. For example, in Patent Document 1, a specific calculation procedure of a residual stress by the cos α method is described.

In an X-ray diffraction system disclosed in Patent Document 1, an X-ray diffraction apparatus is stopped at a given measurement portion on a rail to perform irradiation with X-rays, diffracted X-rays are detected by an imaging plate, and a residual stress is evaluated based on a diffraction ring formed by the diffracted X-rays (Paragraph 0025). The X-ray diffraction system in Patent Document 1 can monitor aged deterioration of each part of the rail by accumulating measurement data at each measurement point on the rail while moving a vehicle equipped with the X-ray diffraction apparatus and by evaluating an average value of the measurement data at each measurement point (Paragraphs 0057 and 0059).

In the meantime, a cast and forged steel product may internally have a locally uneven distribution of chemical components depending on production conditions such as a kind of an element contained therein, a concentration of an element contained therein, a cooling rate in solidification of molten steel, and the like. In this case, the cast and forged steel product tends to be not completely uniform in structure and hardness, and a residual stress occurring inside the cast and forged steel product also tends to vary locally. This tendency is particularly significant in a large cast and forged steel product.

In the case of performing an X-ray residual stress measurement using a cast and forged steel product as a specimen, when a non-uniform portion in the cast and forged steel product is selected as a measurement position, a measurement result of the residual stress may include a large error. Hence, there is a demand for a method for measuring a residual stress, which can properly evaluate a residual stress occurring inside a cast and forged steel product.

### [PRIOR ART DOCUMENTS]

### [PATENT DOCUMENTS]

Patent Document 1: Japanese Unexamined Patent Application Publication No. 2005-241308

### [SUMMARY OF THE INVENTION]

### [PROBLEMS TO BE SOLVED BY THE INVENTION]

The present invention is made in view of the foregoing circumstances, and an object of the present invention is to provide a method for measuring a residual stress which can properly evaluate a residual stress occurring inside a cast and forged steel product.

### [MEANS FOR SOLVING THE PROBLEMS]

An embodiment of the present invention made to solve the above problems is a method for measuring a residual stress in a cast and forged steel product, the method using X-rays, including: irradiating a cast and forged steel product with X-rays; two-dimensionally detecting intensities of diffracted X-rays originating from the X-rays; and calculating a residual stress based on a diffraction ring formed by an intensity distribution of the diffracted X-rays detected in the detecting, wherein the irradiating includes changing a condition for irradiation of the cast and forged steel product with the X-rays, the irradiating is a step of performing the changing each time the cast and forged steel product is irradiated with the X-rays, the calculating is a step of calculating the residual stress each time the cast and forged steel product is irradiated with the X-rays, and the method for measuring a residual stress further includes averaging a plurality of residual stresses calculated in the calculating after the irradiating, the detecting, and the calculating are performed in this order a plurality of times.

In the method for measuring a residual stress, the condition for the irradiation of the cast and forged steel product with the X-rays is changed, and an averaged residual stress is calculated in accordance with the change in the condition for the irradiation with the X-rays; therefore, even when a region including a non-uniform portion in the cast and forged steel product is irradiated with the X-rays, an influence of the non-uniform portion on a calculation result of the residual stress can be reduced. Thus, the method for measuring a residual stress can properly evaluate the residual stress in the cast and forged steel product. In particular, in the method for measuring a residual stress, the residual stress is calculated each time the irradiation with the X-rays is performed and a plurality of residual stresses calculated is averaged, whereby the residual stress can be checked each time the irradiation with the X-rays is performed and the averaged residual stress can be properly calculated.

Another embodiment of the present invention made to solve the above problems is a method for measuring a residual stress in a cast and forged steel product, the method using X-rays, including: irradiating a cast and forged steel product with X-rays; two-dimensionally detecting intensities of diffracted X-rays originating from the X-rays; and calculating a residual stress based on a diffraction ring formed by an intensity distribution of the diffracted X-rays detected in the detecting, wherein the irradiating includes changing a condition for irradiation of the cast and forged steel product with the X-rays, the irradiating is a step of performing the changing each time the cast and forged steel product is irradiated with the X-rays, and, after the irradiating and the detecting are alternately performed a plurality of times, the residual stress is calculated in the calculating based on the diffraction ring formed by the intensity distribution, in which a plurality of the intensities of the diffracted X-rays has been averaged.

In the method for measuring a residual stress, the condition for the irradiation of the cast and forged steel product with the X-rays is changed, and an averaged residual stress is calculated in accordance with the change in the condition for the irradiation with the X-rays; therefore, even when a region including a non-uniform portion in the cast and forged steel product is irradiated with the X-rays, an influence of the non-uniform portion on a calculation result of the residual stress can be reduced. Thus, the method for measuring a residual stress can properly evaluate the residual stress in the cast and forged steel product. In particular, in the method for measuring a residual stress, the intensities of the diffracted X-rays obtained by a plurality of times of irradiation with the X-rays are averaged, and the residual stress is calculated based on the diffraction ring formed by the intensity distribution, in which the intensities have been averaged, whereby the residual stress can be evaluated in a shorter time than that in the case of calculating the residual stress for each diffraction ring.

Still another embodiment of the present invention made to solve the above problems is a method for measuring a residual stress in a cast and forged steel product, the method using X-rays, including: irradiating a cast and forged steel product with X-rays; two-dimensionally detecting intensities of diffracted X-rays originating from the X-rays; and calculating a residual stress based on a diffraction ring formed by an intensity distribution of the diffracted X-rays detected in the detecting, wherein the irradiating includes changing a condition for irradiation of the cast and forged steel product with the X-rays, the irradiating is a step of performing a continuous irradiation with the X-rays while performing the changing, and the residual stress is calculated in the calculating based on the diffraction ring formed by the intensity distribution of the diffracted X-rays, the intensities of the intensity distribution having been averaged by the continuous irradiation with the X-rays.

In the method for measuring a residual stress, the condition for the irradiation of the cast and forged steel product with the X-rays is changed, and an averaged residual stress is calculated in accordance with the change in the condition for the irradiation with the X-rays; therefore, even when a region including a non-uniform portion in the cast and forged steel product is irradiated with the X-rays, an influence of the non-uniform portion on a calculation result of the residual stress can be reduced. Thus, the method for measuring a residual stress can properly evaluate the residual stress in the cast and forged steel product. In particular, in the method for measuring a residual stress, a continuous irradiation with the X-rays is performed while the condition for the irradiation with the X-rays is changed, whereby the residual stress can be evaluated in a shorter time than that in the case of changing the condition for the irradiation with the X-rays each time the irradiation with the X-rays is performed.

In the changing of the method for measuring a residual stress, the condition for the irradiation of the cast and forged steel product with the X-rays is preferably changed so that an X-ray irradiation area is greater than or equal to 20 mm² in total. Consequently, the method for measuring a residual stress can more properly evaluate the residual stress in the cast and forged steel product.

### [EFFECTS OF THE INVENTION]

The method for measuring a residual stress of the present invention can properly evaluate a residual stress in a cast and forged steel product.

### [BRIEF DESCRIPTION OF THE DRAWINGS]

Fig. 1 is a flow chart showing a method for measuring a residual stress of a first embodiment of the present invention.
Fig. 2 is a flow chart showing a method for measuring a residual stress of a second embodiment of the present invention.
Fig. 3 is a flow chart showing a method for measuring a residual stress of a third embodiment of the present invention.
Fig. 4 is a graph showing a relation between a conventional stress and an X-ray stress measured using a test specimen with much segregation.
Fig. 5 is a graph showing a relation between a conventional stress and an X-ray stress measured using a test specimen with little segregation.
Fig. 6 is a graph showing a relation between an X-ray irradiation area and a measurement error of an X-ray stress in the case of changing an X-ray irradiation position.
Fig. 7 is a graph showing a relation between an X-ray irradiation area and a measurement error of an X-ray stress in the case of changing an X-ray irradiation angle.
Fig. 8 is a graph showing a relation between an X-ray irradiation area and a maximum error of an X-ray stress in the case of changing an irradiation position during irradiation with X-rays.

### [DESCRIPTION OF EMBODIMENTS]

Hereinafter, embodiments of the method for measuring a residual stress of the present invention will be described in detail with reference to the drawings.

### First Embodiment

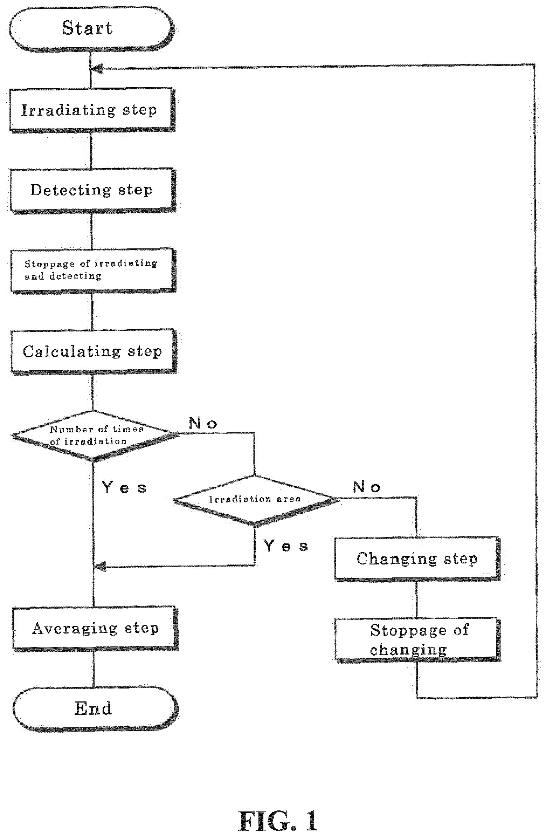

A method for measuring a residual stress shown in Fig. 1 is a method for measuring a residual stress in a cast and forged steel product, the method using X-rays, including: irradiating a cast and forged steel product with X-rays (an irradiation step), two-dimensionally detecting intensities of diffracted X-rays originating from the X-rays (a detection step), and calculating a residual stress based on a diffraction ring formed by an intensity distribution of the diffracted X-rays detected in the detection step (a calculation step). The irradiation step includes changing a condition for irradiation of the cast and forged steel product with the X-rays (a changing step), and the changing step is performed each time the cast and forged steel product is irradiated with the X-rays. In the calculation step, the residual stress is calculated each time the cast and forged steel product is irradiated with the X-rays. The method for measuring a residual stress further includes, after the irradiation step, the detection step, and the calculation step are performed in this order a plurality of times, averaging a plurality of residual stresses calculated in the calculation step (an averaging step).

In the method for measuring a residual stress, an X-ray irradiation apparatus and an X-ray stress measurement apparatus including a two-dimensional detector are used. In the method for measuring a residual stress, the condition for the irradiation of the cast and forged steel product with the X-rays is changed each time the cast and forged steel product is irradiated with the X-rays, and the residual stress is calculated each time the cast and forged steel product is irradiated with the X-rays. Then, in the method for measuring a residual stress, in the case where a number of times of irradiation with the X-rays or an X-ray irradiation area in total has reached a certain value, an averaged residual stress of the residual stresses is calculated. In other words, in the method for measuring a residual stress, the cast and forged steel product is irradiated with the X-rays under a variety of conditions for the irradiation and the residual stresses calculated for each condition for the irradiation are averaged.

### Irradiation Step

The irradiation step is a step of irradiating the cast and forged steel product with the X-rays from the X-ray irradiation apparatus and includes the changing step of changing the condition for the irradiation of the cast and forged steel product with the X-rays. In the irradiation step, the cast and forged steel product is irradiated with the X-rays without changing the condition for the irradiation in one irradiation with the X-rays, and the condition for the irradiation with the X-rays is changed when the changing step is performed.

### Changing Step

The changing step is a step of changing the condition for the irradiation with the X-rays by changing an X-ray irradiation position, an X-ray irradiation angle, or an X-ray irradiation area with respect to the cast and forged steel product. The changing step is performed for each irradiation with the X-rays under a condition where the number of times of irradiation with the X-rays or the X-ray irradiation area in a plurality of times of irradiation with the X-rays has not reached a prescribed value. It is to be noted that, in the case where there is a possibility that a residual stress occurring inside the cast and forged steel product largely differs depending on a portion, a distance by which the X-ray irradiation position is changed for each irradiation with the X-rays preferably falls, but not particular limited to, for example, within five times an X-ray irradiation diameter.

The X-ray irradiation angle is an incidence angle of the X-rays on the cast and forged steel product and is changed by rotating an irradiation port of the X-ray irradiation apparatus relatively to the cast and forged steel product with the X-ray irradiation position fixed. The X-ray irradiation position is changed by moving the irradiation port of the X-ray irradiation apparatus relatively to the cast and forged steel product with the X-ray irradiation angle fixed. The X-ray irradiation area is an area of an X-ray irradiation region on a surface of the cast and forged steel product and is changed, for example, by changing the irradiation angle, changing a distance between the irradiation port of the X-ray irradiation apparatus and the cast and forged steel product, changing a collimator diameter of the X-ray irradiation apparatus, or the like.

### Detection Step

The detection step is a step of detecting, by a two-dimensional detector, the intensities of the diffracted X-rays originating from the X-rays with which the cast and forged steel product is irradiated. The cast and forged steel product is polycrystalline; therefore, the X-rays with which the cast and forged steel product is irradiated are diffracted by a large number of crystals at angles that satisfy the Bragg's diffraction condition. The X-rays diffracted by the large number of crystals are detected as diffracted X-rays by the two-dimensional detector. The two-dimensional detector detects the intensities of the diffracted X-rays, and an intensity distribution of the diffracted X-rays forms a diffraction ring.

### Calculation Step

The calculation step is a step of calculating, each time the cast and forged steel product is irradiated with the X-rays, the residual stress based on the diffraction ring formed by the intensity distribution of the diffracted X-rays detected by the two-dimensional detector. As a method for calculating the residual stress based on the diffraction ring, a calculation method of a cos α method is used; however, a method in which a stress is directly calculated from an X-ray distortion may also be used.

### Averaging Step

The averaging step is a step of averaging the plurality of the residual stresses calculated in the calculation step and is performed after a plurality of times of irradiation with the X-rays is completed.

The steps of the method for measuring a residual stress are performed by the following procedure. First, in the method for measuring a residual stress, the irradiation step and the detection step are performed, the irradiation with the X-rays and the detection of the X-rays are stopped, and then, the calculation step is performed. When the residual stress is calculated based on the diffraction ring in the calculation step, X-ray diffraction information relating to the intensities of the diffracted X-rays in the two-dimensional detector is initialized. In the case where the number of times of irradiation with the X-rays or the X-ray irradiation area in total has not reached a prescribed value after the calculation step is performed, the changing step is performed. In the case of performing the changing step, the change in the condition for the irradiation with the X-rays in the changing step is stopped, and then, the irradiation step and the detection step are performed again. Meanwhile, in the case where the number of times of irradiation with the X-rays or the X-ray irradiation area in total has reached the prescribed value, the plurality of the residual stresses calculated is averaged in the averaging step.

### Advantages

In the method for measuring a residual stress, the condition for the irradiation of the cast and forged steel product with the X-rays is changed, and an averaged residual stress is calculated in accordance with the change in the condition for the irradiation with the X-rays; therefore, even in the case of irradiating a region including a non-uniform portion in the cast and forged steel product with the X-rays, an influence of the non-uniform portion on a calculation result of the residual stress can be reduced. In particular, in the method for measuring a residual stress, the residual stress is calculated each time the irradiation with the X-rays is performed, and the plurality of the residual stresses calculated is averaged; therefore, a residual stress according to the change in the condition for the irradiation with the X-rays can be checked, and the averaged residual stress can be property calculated.

Furthermore, in the method for measuring a residual stress, the condition for the irradiation with the X-rays is changed by changing the X-ray irradiation position, the X-ray irradiation angle, or the X-ray irradiation area with respect to the cast and forged steel product in the changing step. By changing the X-ray irradiation position or the X-ray irradiation area, residual stresses of a variety of portions of the cast and forged steel product can be evaluated; by changing the X-ray irradiation angle, residual stresses can be evaluated based on various orientations of crystals that contribute to diffraction. Thus, the method for measuring a residual stress can properly evaluate a residual stress in a cast and forged steel product by irradiating the cast and forged steel product with X-rays under a variety of conditions for the irradiation.

### Second Embodiment

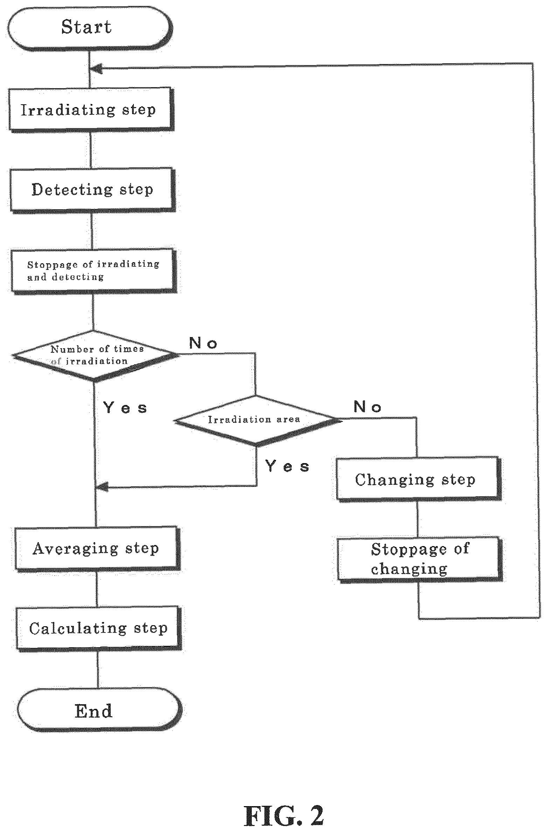

A method for measuring a residual stress shown in Fig. 2 is different from the method for measuring a residual stress of the first embodiment in that an intensity distribution of diffracted X-rays obtained by a plurality of times of irradiation with X-rays is averaged and a residual stress is calculated based on a diffraction ring formed by the intensity distribution, which has been averaged. The method for measuring a residual stress of the second embodiment is similar to the method for measuring a residual stress of the first embodiment in terms of the irradiation step and the detection step and is different from the method for measuring a residual stress of the first embodiment in terms of the calculation step and the averaging step.

The method for measuring a residual stress is a method for measuring a residual stress in a cast and forged steel product, the method using X-rays, including: irradiating a cast and forged steel product with X-rays (an irradiation step), two-dimensionally detecting intensities of diffracted X-rays originating from the X-rays (a detection step), and calculating a residual stress based on a diffraction ring formed by an intensity distribution of the diffracted X-rays detected in the detection step (a calculation step). The irradiation step includes changing a condition for irradiation of the cast and forged steel product with the X-rays (a changing step), and the changing step is performed each time the cast and forged steel product is irradiated with the X-rays. The method for measuring a residual stress further includes, after the irradiation step and the detection step are performed in this order a plurality of times, averaging a plurality of the intensities of the diffracted X-rays detected in the detection step (an averaging step). Then, in the calculation step of the method for measuring a residual stress, the residual stress is calculated based on the diffraction ring formed by the intensity distribution, in which the plurality of the intensities of the diffracted X-rays has been averaged.

In the method for measuring a residual stress, the condition for the irradiation of the cast and forged steel product with the X-rays is changed each time the cast and forged steel product is irradiated with the X-rays, and the intensities of the diffracted X-rays are detected each time the cast and forged steel product is irradiated with the X-rays. In the method for measuring a residual stress, in the case where a number of times of irradiation with the X-rays or an X-ray irradiation area in total has reached a certain value, the plurality of the intensities of the diffracted X-rays, which have been detected, is averaged. Then, in the method for measuring a residual stress, the residual stress is calculated based on the diffraction ring formed by the intensity distribution, which has been averaged. In other words, in the method for measuring a residual stress, the cast and forged steel product is irradiated with the X-rays under a variety of conditions for the irradiation, and residual stresses are averaged by averaging the intensities of the diffracted X-rays detected for each condition for the irradiation.

### Averaging Step

The averaging step is a step of averaging the plurality of the intensities of the diffracted X-rays detected in the detection step and is performed after a plurality of times of irradiation with the X-rays is completed.

### Calculation Step

The calculation step is a step of calculating the residual stress based on the diffraction ring formed by the intensity distribution of the diffracted X-rays, which has been averaged in the averaging step. As a method for calculating the residual stress based on the diffraction ring, a cos α method is used; however, a method in which a stress is directly calculated from an X-ray distortion may also be used.

The steps of the method for measuring a residual stress are performed by the following procedure. First, in the method for measuring a residual stress, the irradiation step and the detection step are performed, and the irradiation with the X-rays and the detection of the X-rays are stopped. In the case where the number of times of irradiation with the X-rays or the X-ray irradiation area in total has not reached a prescribed value, the changing step is performed. In the case of performing the changing step, the change in the condition for the irradiation with the X-rays in the changing step is stopped, and then, the irradiation step and the detection step are performed again. X-ray diffraction information relating to the intensities of the diffracted X-rays in a two-dimensional detector is extracted from the two-dimensional detector for each irradiation with the X-rays and retained separately. Furthermore, when the X-ray diffraction information is extracted from the two-dimensional detector, the X-ray diffraction information in the two-dimensional detector is initialized. Meanwhile, in the case where the number of times of irradiation with the X-rays or the X-ray irradiation area in total has reached the prescribed value, the plurality of the intensities of the diffracted X-rays is averaged in the averaging step based on a plurality of pieces of the X-ray diffraction information retained, and then, the residual stress is calculated in the calculation step.

It is to be noted that, in the method for measuring a residual stress, the intensity distribution of the plurality of the diffracted X-rays may be averaged by a procedure in which pieces of the X-ray diffraction information in the two-dimensional detector are superposed on one another without being initialized for each irradiation with the X-rays. In this case, since an averaged X-ray diffraction information can be obtained from the two-dimensional detector when the plurality of the times of irradiation with the X-rays is completed, the averaging step can be omitted from the method for measuring a residual stress.

### Advantages

In the method for measuring a residual stress, the condition for the irradiation of the cast and forged steel product with the X-rays is changed and an averaged residual stress is calculated in accordance with the change in the condition for the irradiation with the X-rays; therefore, even in the case of irradiating a region including a non-uniform portion in the cast and forged steel product with the X-rays, an influence of the non-uniform portion on a calculation result of the residual stress can be reduced. In particular, in the method for measuring a residual stress, the intensities of the diffracted X-rays obtained by the plurality of the times of irradiation with the X-rays are averaged, and the residual stress is calculated based on the diffraction ring formed by the intensity distribution, which has been averaged; therefore, the residual stress can be evaluated in a shorter time than that in the method for measuring a residual stress of the first embodiment, in which the residual stress is calculated for each irradiation with the X-rays.

### Third Embodiment

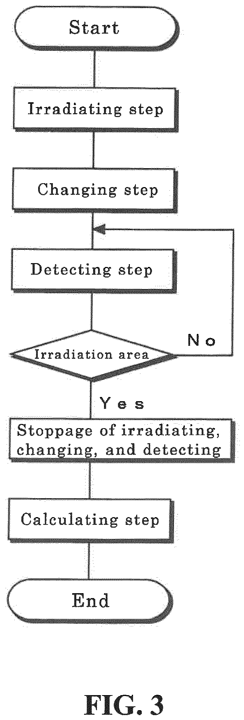

A method for measuring a residual stress shown in Fig. 3 is different from the method for measuring a residual stress of the second embodiment in that a continuous irradiation with X-rays is performed while a condition for irradiation with the X-rays is changed. The method for measuring a residual stress of the third embodiment is similar to the method for measuring a residual stress of the second embodiment in terms of the detection step and the calculation step and is different from the method for measuring a residual stress of the second embodiment in terms of the irradiation step. Furthermore, the method for measuring a residual stress of the third embodiment does not include the averaging step, which is another difference from the method for measuring a residual stress of the second embodiment.

The method for measuring a residual stress is a method for measuring a residual stress in a cast and forged steel product, the method using X-rays, including: irradiating a cast and forged steel product with X-rays (an irradiation step), two-dimensionally detecting intensities of diffracted X-rays originating from the X-rays (a detection step), and calculating a residual stress based on a diffraction ring formed by an intensity distribution of the diffracted X-rays detected in the detection step (a calculation step). The irradiation step includes changing a condition for irradiation of the cast and forged steel product with the X-rays (a changing step), and a continuous irradiation with the X-rays is performed while the changing step is performed. Then, in the calculation step of the method for measuring a residual stress, the residual stress is calculated based on the diffraction ring formed by the intensity distribution of the diffracted X-rays, wherein the intensities of the intensity distribution have been averaged by the continuous irradiation with the X-rays.

In the method for measuring a residual stress, the cast and forged steel product is continuously irradiated with the X-rays while the condition for the irradiation with the X-rays is changed, and at the same time, the intensities of the diffracted X-rays are detected. That is to say, in the method for measuring a residual stress, the intensities of the diffracted X-rays averaged in accordance with the change in the condition for the irradiation with the X-rays are detected. Then, in the method for measuring a residual stress, in the case where an X-ray irradiation area in total has reached a certain value, the residual stress is calculated based on the diffraction ring formed by the intensity distribution, which has been averaged. In other words, in the method for measuring a residual stress, the cast and forged steel product is continuously irradiated with the X-rays while the condition for the irradiation is changed, and the intensities of the diffracted X-rays, which have been detected, are averaged, whereby residual stresses are averaged.

### Irradiation Step

The irradiation step is a step of irradiating the cast and forged steel product with X-rays from an X-ray irradiation apparatus and includes the changing step of changing the condition for the irradiation of the cast and forged steel product with the X-rays. In the irradiation step, the cast and forged steel product is continuously irradiated with the X-rays while the condition for irradiation is changed. In other words, in the irradiation step, the irradiation with the X-rays is performed while the changing step is performed. The changing step is similar to the changing step of the method for measuring a residual stress of the first embodiment, wherein an X-ray irradiation position, an X-ray irradiation angle, or an X-ray irradiation area with respect to the cast and forged steel product is changed.

The steps of the method for measuring a residual stress are performed by the following procedure. First, in the method for measuring a residual stress, the irradiation step of irradiating with the X-rays is performed while the changing step is performed. The detection step is performed in synchronization with the irradiation step. In the case where the X-ray irradiation area in total has not reached a prescribed value, the irradiation step and the detection step are continued. In the meantime, pieces of X-ray diffraction information relating to the intensities of the diffracted X-rays in a two-dimensional detector are superposed on one another without being initialized. Meanwhile, when the X-ray irradiation area in total has reached the prescribed value, the residual stress is calculated in the calculation step.

### Advantages

In the method for measuring a residual stress, the condition for the irradiation of the cast and forged steel product with the X-rays is changed, and an averaged residual stress is calculated in accordance with the change in the condition for the irradiation with the X-rays; therefore, even in the case of irradiating a region including a non-uniform portion in the cast and forged steel product with the X-rays, an influence of the non-uniform portion on a calculation result of the residual stress can be reduced. In particular, in the method for measuring a residual stress, the continuous irradiation with the X-rays is performed while the condition for the irradiation with the X-rays is changed; therefore, the residual stress can be evaluated in a shorter time than that in the method for measuring a residual stress of the second embodiment, in which the condition for the irradiation with the X-rays is changed for each irradiation with the X-rays.

### Other Embodiments

The method for measuring a residual stress of the present invention is not limited to the above embodiments.

In the first embodiment and the second embodiment, the methods have been described in which, in the case where the number of times of irradiation with the X-rays or the X-ray irradiation area in total has reached the prescribed value, an averaged residual stress is calculated; in the third embodiment, the method has been described in which, in the case where the X-ray irradiation area in total has reached the prescribed value, an averaged residual stress is calculated; however, in light of reducing the influence of the non-uniform portion in the cast and forged steel product, the averaged residual stress is preferably calculated in the case where the X-ray irradiation area in total is greater than or equal to a predetermined lower limit. In other words, the condition for the irradiation of the cast and forged steel product with the X-rays is preferably changed so that the X-ray irradiation area in total is enough for averaging. In this case, the lower limit of the X-ray irradiation area in total is preferably 20 mm², more preferably 23 mm², and still more preferably 26 mm². If the X-ray irradiation area in total is less than the lower limit, the influence of the non-uniform portion in the cast and forged steel product may fail to be reduced.

Furthermore, the changing step in which the condition for the irradiation with the X-rays is changed by changing the X-ray irradiation position, the X-ray irradiation angle, or the X-ray irradiation area with respect to the cast and forged steel product has been described in the above embodiments; however, at least one of the irradiation position, the irradiation angle, and the irradiation area may be changed in the changing step; for example, the irradiation position and the irradiation angle may be changed at the same time.

### [EXAMPLES]

Hereinafter, the present invention will be described more in detail by way of Examples; however, the present invention is not limited to the Examples.

### Tension Test of Test Specimen

First, a test specimen with much segregation and a test specimen with little segregation were cut out from a large cast and forged steel product having a weight of greater than 1 t. As the large cast and forged steel product, chromium-molybdenum-based alloy steel having a bainite structure was used. Furthermore, on the basis of knowledge that a black line is observed in a portion with much segregation of a cast and forged steel product, a portion with much segregation and a portion with little segregation were distinguished from each other by use of a macro-texture photo of the large cast and forged steel product. Furthermore, as the test specimens, rods each including a plate-shaped portion with a length of 70 mm, a width of 12.5 mm, and a thickness of 3 mm in a middle were cut out, and the plate-shaped portions of the test specimens, which had been cut out, were electrolytically polished to a thickness of approximately 0.1 mm.

A test was carried out in which a tension tester was used and, in a state where a tensile stress was applied in a longitudinal direction of each of two kinds of test specimens (the test specimen with much segregation and the test specimen with little segregation), a conventional stress obtained from a load cell of the tension tester was compared with a residual stress measured using X-rays (hereinafter, referred to as "X-ray stress"). Furthermore, as measurement positions of the X-ray stress, 3 × 3, i.e., nine points were set at regular intervals in a 6 mm × 6 mm region of the plate-shaped portion of each of the test specimens, which had been electrolytically polished.

Chromium Kα rays were used as the X-rays, a collimator diameter was set to 1.0 mm, an X-ray irradiation distance was set to 80 mm, an X-ray irradiation angle with respect to the test specimens was set to 35°, and an X-ray irradiation area was set to approximately 6.5 mm². Furthermore, diffracted X-rays from a (211) plane of iron were detected by a two-dimensional detector. As an X-ray elastic constant used for calculation of a residual stress from an obtained diffraction ring, a standard one employed for a steel material was used. Specifically, a Young's modulus E and a Poisson's ratio v, which were used for calculation of the X-ray elastic constant, were set to 224 GPa and 0.28, respectively.

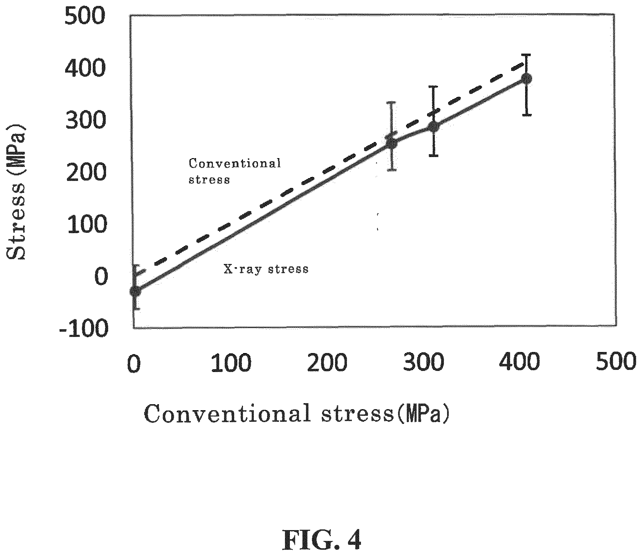

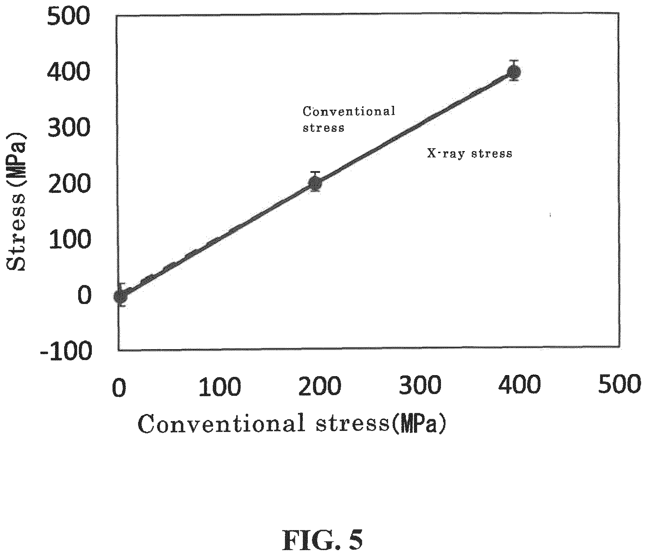

An X-ray stress of the test specimen with much segregation was measured at conventional stresses of 0 MPa, 269 MPa, 312 MPa, and 409 MPa. Furthermore, an X-ray stress of the test specimen with little segregation was measured at conventional stresses of 0 MPa, 197 MPa, and 396 MPa. Fig. 4 is a graph showing a relation between the conventional stress and the X-ray stress measured using the test specimen with much segregation, and Fig. 5 is a graph showing a relation between the conventional stress and the X-ray stress measured using the test specimen with little segregation. It is to be noted that solid lines in the graphs each represent an average value of X-ray stresses at the nine measurement positions, dashed lines in the graphs each represent the conventional stress, and ends of a line extending vertically represent a maximum value and a minimum value of the X-ray stresses at the nine measurement positions.

As shown in Fig. 4, a difference between the maximum value and the minimum value of the X-ray stress in the test specimen with much segregation was approximately 80 MPa at the conventional stress of 0 MPa and was greater than or equal to 100 MPa at the conventional stresses other than 0 MPa, and the difference was confirmed to be very large in either case. For further examination, a percentage obtained by dividing the difference between the maximum value and the minimum value of the X-ray stress by the conventional stress was defined as a measurement error of the X-ray stress; the measurement error in the test specimen with much segregation was approximately 49% at the conventional stress of 269 MPa and was confirmed to be a very large value. Furthermore, as shown in Fig. 5, it was confirmed that the difference between the maximum value and the minimum value of the X-ray stress in the test specimen with little segregation was not small, either. A measurement error of the X-ray stress in the test specimen with little segregation was also examined; the measurement error was approximately 17% at the conventional stress of 197 MPa and was confirmed to be not small.

Meanwhile, both in the test specimen with much segregation and the test specimen with little segregation, it was confirmed that the average value of the X-ray stresses at the nine points was in proximity to the value of the conventional stress. This indicates that, by averaging a plurality of X-ray stresses obtained by changing the X-ray irradiation position, an influence of a non-uniform portion in a cast and forged steel product on calculation of the X-ray stress can be reduced.

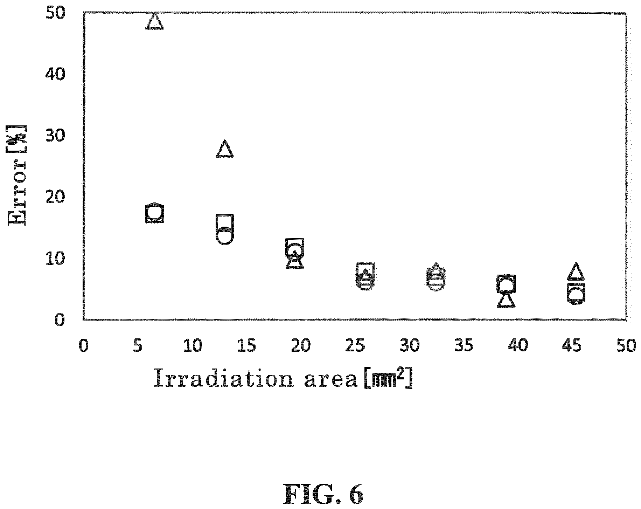

Next, the X-ray irradiation area was increased to examine a change in the difference between the maximum value and the minimum value of the X-ray stress. In the examination, in addition to the two kinds of test specimens described above, a test specimen cut out from a portion with little segregation of nickel-chromium-molybdenum-based alloy steel having a martensite structure was used. As described above, an area for one irradiation with X-rays was approximately 6.5 mm², and the X-ray irradiation area was increased by changing the X-ray irradiation position. Fig. 6 is a graph showing a relation between an X-ray irradiation area in total and a measurement error of an X-ray stress. In Fig. 6, a triangle mark represents data of the test specimen with much segregation having the bainite structure, a square mark represents data of the test specimen with little segregation having the bainite structure, and a circle mark represents data of the test specimen with little segregation having the martensite structure. It is to be noted that "a measurement error of an X-ray stress" refers to a percentage obtained by dividing the difference between the maximum value and the minimum value of the X-ray stress by the conventional stress.

As shown in Fig. 6, in all the test specimens, the measurement error of the X-ray stress tended to decrease with an increase in the X-ray irradiation area in total. Furthermore, it was confirmed that the measurement error of the X-ray stress sufficiently decreased when the X-ray irradiation area reached 20 mm² in total and the measurement error of the X-ray stress was less than or equal to 10% when the X-ray irradiation area was greater than or equal to 26 mm² in total.

### Bending Test of Test Specimen

A four-point bending test was carried out using the above-described test specimen with little segregation having the bainite structure. The test specimen used for the four-point bending test had a plate shape with a length of 150 mm, a width of 20 mm, and a thickness of 3 mm. A central portion of the test specimen was electrolytically polished to a thickness of approximately 0.1 mm.

A test was carried out in which a four-point bending tester was used and, in a state where a bending stress was applied in a thickness direction of the test specimen, a conventional stress obtained from a load cell of the four-point bending tester was compared with an X-ray stress. Chromium Kα rays were used as the X-rays, a collimator diameter was set to 1.0 mm, and an X-ray irradiation distance was set to 80 mm. Furthermore, diffracted X-rays from a (211) plane of iron were detected by a two-dimensional detector. An X-ray elastic constant used for calculation of a residual stress from an obtained diffraction ring was identical to that used in the tension test.

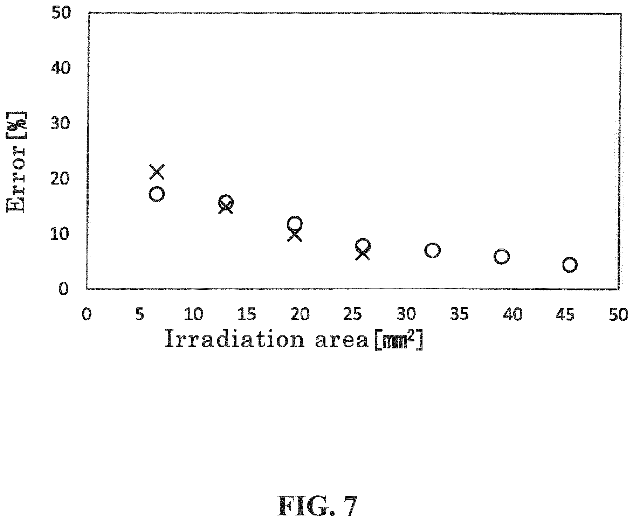

The four-point bending test was performed in the following manner: the central portion of the test specimen, which had been electrolytically polished, was irradiated with X-rays, wherein an X-ray irradiation angle with respect to the test specimen was set to 35° and an X-ray irradiation area was set to approximately 6.5 mm², and then, the irradiation angle was reduced by 5° for each irradiation with the X-rays. Fig. 7 is a graph showing a relation between an X-ray irradiation area in total and a measurement error of an X-ray stress. In Fig. 7, a cross mark represents data of the measurement error in the case of increasing the total irradiation area while changing the X-ray irradiation angle from 35° to 20°, and a circle mark represents data of the measurement error in the test specimen identical to the one used in the tension test in Fig. 6, in which the X-ray irradiation position was changed.

As shown in Fig. 7, also in the case of changing the X-ray irradiation angle with respect to the test specimen, the measurement error of the X-ray stress tended to decrease with an increase in the X-ray irradiation area in total. Furthermore, it was confirmed that the measurement error of the X-ray stress sufficiently decreased when the X-ray irradiation area reached 20 mm² in total and the measurement error of the X-ray stress decreased to approximately 7% when the X-ray irradiation area was greater than or equal to 26 mm² in total.

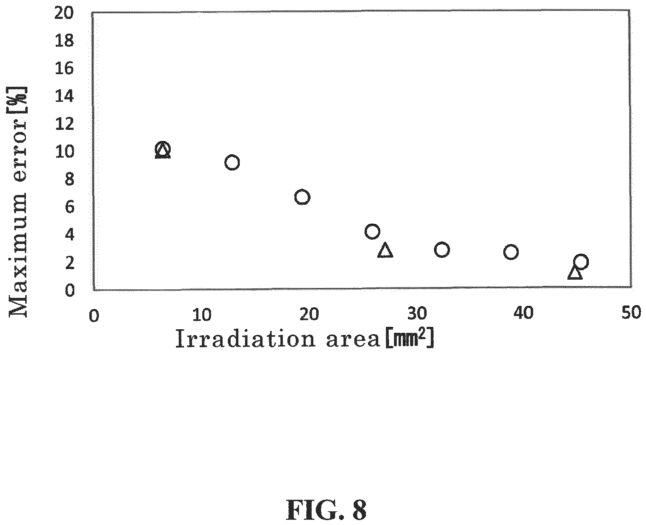

Furthermore, as another four-point bending test, a test was carried out in which the X-ray irradiation position was changed by moving the test specimen while irradiating the test specimen with the X-rays. In this test, the X-ray irradiation position was changed so that the X-ray irradiation area accounted for 6.5 mm², 27.1 mm², and 44.8 mm² in total. Fig. 8 is a graph showing a relation between the X-ray irradiation area in total and a maximum error of an X-ray stress. In Fig. 8, a triangle mark represents data of the maximum error in the case of increasing the total irradiation area by changing the X-ray irradiation position during the irradiation with the X-rays, and a circle mark represents data of the maximum error in the test specimen identical to the one used in the tension test in Fig. 6, in which the X-ray irradiation position was changed. It is to be noted that "a maximum error of an X-ray stress" refers to a percentage obtained by dividing a difference between the maximum value of the X-ray stress and the conventional stress by the conventional stress and corresponds to about a half of the above-described measurement error of the X-ray stress.

As shown in Fig. 8, also in the case of moving the test specimen while irradiating the test specimen with the X-rays, the maximum error of the X-ray stress tended to decrease with an increase in the X-ray irradiation area in total. Furthermore, it was confirmed that the maximum error of the X-ray stress decreased to less than or equal to 5% when the X-ray irradiation area reached 27.1 mm² in total. When converted into a measurement error, the maximum error corresponds to a measurement error of less than or equal to 10%.

### [INDUSTRIAL APPLICABILITY]

The method for measuring a residual stress of the present invention can properly evaluate a residual stress in a cast and forged steel product.

## Claims

1. A method for measuring a residual stress in a cast and forged steel product, the method using X-rays, comprising:
irradiating a cast and forged steel product with X-rays;
two-dimensionally detecting intensities of diffracted X-rays originating from the X-rays; and
calculating a residual stress based on a diffraction ring formed by an intensity distribution of the diffracted X-rays detected in the detecting, wherein
the irradiating comprises changing a condition for irradiation of the cast and forged steel product with the X-rays,
the irradiating is a step of performing the changing each time the cast and forged steel product is irradiated with the X-rays,
the calculating is a step of calculating the residual stress each time the cast and forged steel product is irradiated with the X-rays, and
the method for measuring a residual stress further comprises averaging a plurality of residual stresses calculated in the calculating after the irradiating, the detecting, and the calculating have been performed in this order a plurality of times.

2. A method for measuring a residual stress in a cast and forged steel product, the method using X-rays, comprising:
irradiating a cast and forged steel product with X-rays;
two-dimensionally detecting intensities of diffracted X-rays originating from the X-rays; and
calculating a residual stress based on a diffraction ring formed by an intensity distribution of the diffracted X-rays detected in the detecting, wherein
the irradiating comprises changing a condition for irradiation of the cast and forged steel product with the X-rays,
the irradiating is a step of performing the changing each time the cast and forged steel product is irradiated with the X-rays, and
after the irradiating and the detecting have been alternately performed a plurality of times, the residual stress is calculated in the calculating based on the diffraction ring formed by the intensity distribution, in which a plurality of the intensities of the diffracted X-rays have been averaged.

3. A method for measuring a residual stress in a cast and forged steel product, the method using X-rays, comprising:
irradiating a cast and forged steel product with X-rays;
two-dimensionally detecting intensities of diffracted X-rays originating from the X-rays; and
calculating a residual stress based on a diffraction ring formed by an intensity distribution of the diffracted X-rays detected in the detecting, wherein
the irradiating comprises changing a condition for irradiation of the cast and forged steel product with the X-rays,
the irradiating is a step of performing continuous irradiation with the X-rays while performing the changing, and
the residual stress is calculated in the calculating based on the diffraction ring formed by the intensity distribution of the diffracted X-rays, the intensities of the intensity distribution having been averaged by the continuous irradiation with the X-rays.

4. The method for measuring a residual stress according to any one of claims 1 to 3, wherein the condition for the irradiation of the cast and forged steel product with the X-rays is changed in the changing so that an X-ray irradiation area is greater than or equal to 20 mm² in total.
